# EUROPEAN PATENT APPLICATION

(11) **EP 3 760 156 A1**
(43) Date of publication of application: **06.01.2021**
(21) Application number: 19184210.3
(22) Date of filing: 03.07.2019
(51) Int. Cl.: A61B 90/00, A61B 90/53, A61B 90/50, G02B 27/01

(54) **DISPLAY DURING MEDICAL EXAMINATIONS**

(71) Applicant: Koninklijke Philips N.V., 5656 AG Eindhoven (NL)
(72) Inventor: CARELSEN, Bart, 5656 AE Eindhoven (NL); VAN ALFEN, Martine, 5656 AE Eindhoven (NL); HOLTHUIZEN, Ronaldus Frederik Johannes, 5656 AE Eindhoven (NL)
(74) Representative: de Haan, Poul Erik

(57) **Abstract**

The present invention relates to displaying during medical examinations. In order to provide a facilitated and more economic and space saving improved device for supporting a user during medical examinations, a display device (10) for use during medical examinations is provided. The display device comprises a support structure (12), a camera arrangement and a display arrangement. The camera arrangement and the display arrangement are attached to the support structure; and the support structure is configured to be carried by a user as a head mounted gear. Further, the camera arrangement comprises at least one optical camera (14) configured to provide live images of a region of interest in front of a user. Still further, the display arrangement comprises at least one projection surface (16) configured to display at least a part of the provided images of the region of interest to the user. Furthermore, the at least one optical camera has a line of vision (*V_{L}*), and the display arrangement is provided for a user's viewing direction (*V_{D}*) during use. The line of vision and the user's viewing direction are arranged angular in relation to each other with an angle in a range of between 30° to 80°.

## Description

### FIELD OF THE INVENTION

The present invention relates to a display device for use during medical examinations, to a medical imaging system and to a method for providing visual information during medical examination.

### BACKGROUND OF THE INVENTION

During medical examinations, for example during surgical operations, a user like a surgeon needs to view the subject, i.e. to look at the location of the subject where the interaction with the subject takes place. For example, surgical loupes attached to the surgeon may be used to provide surgeons with a more detailed way. Further, also operating microscopes may be used. These may be arranged such that they provide an improved ergonomic posture to the surgeons. However, it has been shown that these are costly and are cumbersome to handle.

### SUMMARY OF THE INVENTION

There may thus be a need to provide a facilitated and more economic and space saving improved device for viewing support of a user during medical examinations.

The object of the present invention is solved by the subject-matter of the independent claims; further embodiments are incorporated in the dependent claims. It should be noted that the following described aspects of the invention apply also for the display device for use during medical examinations, for the medical imaging system and for the method for providing visual information during medical examination.

According to the present invention, a display device for use during medical examinations is provided. The display device comprises a support structure, a camera arrangement and a display arrangement. The camera arrangement and the display arrangement are attached to the support structure; and the support structure is configured to be carried by a user as a head mounted gear. Further, the camera arrangement comprises at least one optical camera configured to provide live images of a region of interest in front of the user. The display arrangement comprises at least one projection surface configured to display at least a part of the provided images of the region of interest to the user. The at least one optical camera has a line of vision, and the display arrangement is provided for a user's viewing direction during use. The camera's line of vision and the user's viewing direction are arranged angular in relation to each other with an angle in a range of between 30° to 80°.

As an effect, the angular arrangement of the user's viewing direction and the camera's line of vision provides an ergonomic augmented reality display. Adding a camera to a head mounted augmented reality display where the camera is facing downwards, allows surgeons to see the surgical site while he/she can keep his/her ergonomic upright posture. Thus, an improved ergonomic posture is provided without bulky means like a microscope. By providing a downward facing camera on e.g. augmented reality glasses and projecting the camera image on the augmented reality display in a centric location, a user like a surgeon can maintain a comfortable ergonomic posture while viewing high-quality, detailed images.

According to an example, a hand detector is provided configured to recognize and detect at least a part of a user's hand in the region of interest in front of a user. The images displayed by the display are focused on the detected hand.

This provides that the camera automatically detects the hands and keeps the focus of the display there to allow movement of the head without disturbing the image. Automatic hand recognition also allows to keep the surgical site in view despite movement of the head.

For example, the camera provides a much larger field of view such that the hand region can be selected. In another example, the camera is provided movable such that the hand region can be focused at.

According to an example, a magnifier is provided configured to magnify the images provided by the camera such that the display shows the region of interest in a magnified manner. The magnification is provided such that the displayed image is focused on the user's hands.

This provides that the user is visually focused on the region of interest, e.g. the part of a subject that the user is interacting with, while the camera provides a larger view to ensure that the assigned part of the area covered by the camera is actually magnified.

Adding the magnifier to the downwards facing camera, allows surgeons to see the surgical site magnified and thus in more detail, while he/she can maintain his/her ergonomic upright posture. This relief is particular of interest when focusing on a detailed task is required.

In an option, the hand detector is provided in combination with the magnifier to provide a visual representation to the user of the field of current interaction, when this is defined by where the user's hands are.

According to an example, during use, the camera's line of vision is inclined downwards to a larger extent than the user's viewing direction. Further, during use, the user's viewing direction is in a horizontal range of 25° inclined downwards from the horizontal, and 10° inclined upwards from the horizontal. The line of vision is in a range of 25° to 90° inclined downwards from the horizontal.

According to an example, the at least one projection surface is at least partially transparent such that the user can also at least partly observe reality through the projection surface.

According to an alternative example, the at least one projection surface is a display surface on which the images provided by the camera are presented. Further, for providing a live view of the situation along the viewing direction, further respective image data is provided and displayed on the display surface.

According to an example, the display arrangement comprises a light source and a light transmitter. The light source is provided remote from the support structure and is configured to be carried by the user at the hip, shoulder, back or arm, or combinations thereof. The light transmitter optically couples the light source with the projection surface.

According to the present invention, also a medical imaging system is provided that comprises a display device according to one of the preceding examples. At least one additional imaging device is providing further image data of the region of interest. The further image data is also shown on the display.

According to an example, the at least one additional imaging device comprises a hyperspectral camera and a light source suitable for hyperspectral imaging. As an option, the hyperspectral camera is attached to the display device.

The option of attaching the hyperspectral camera to the display device provides the advantage that the user is not required to manually point a camera towards a region of interest. Due to the attachment, the hyperspectral data is provided for exactly the location which the user is looking at. Thus, hyperspectral information is provided without additional external camera systems. As an effect, hyperspectral imaging provides enhanced anatomical information in addition to the visual images.

According to the present invention, also a method for providing visual information during medical examination is provided. The method comprises the following steps:
a) acquiring live images of a region of interest in front of a user by at least one optical camera of a camera arrangement; and
b) displaying at least a part of the provided images of the region of interest to the user by at least one projection surface of a display arrangement.

The camera arrangement and the display arrangement are attached to the support structure; and the support structure is carried by a user as a head mounted gear. The at least one optical camera has a line of vision, and the display arrangement is provided for a user's viewing direction during use; and the camera's line of vision and the user's viewing direction are arranged angular in relation to each other with an angle in a range of between 30° to 80°.

According to an aspect, in a use position, e.g. a normal use scenario when applied by a surgeon standing next to a subject support, the camera is a downward facing camera. The camera image is projected on the augmented reality display in a centric location, the surgeon can maintain a comfortable ergonomic posture while viewing high-quality detailed images. Surgery and surgical navigation systems are provided as examples for medical application.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a display device for use during medical examinations.
Fig. 2 shows an example of a medical imaging system.
Fig. 3 shows an example of a display device for use during medical examinations in relation with a user and a further person and a subject during a medical examination.
Fig. 4 shows a front view of an example of a display device when applied by a user.
Fig. 5 shows basic steps of an example of a method for providing visual information during medical examination.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

In the following, the term "subject" is used. This term subject may also be referred to as individual. The subject may further also be referred to as patient, although it is noted that this term does not indicate whether any illness or disease is actually present with the subject.

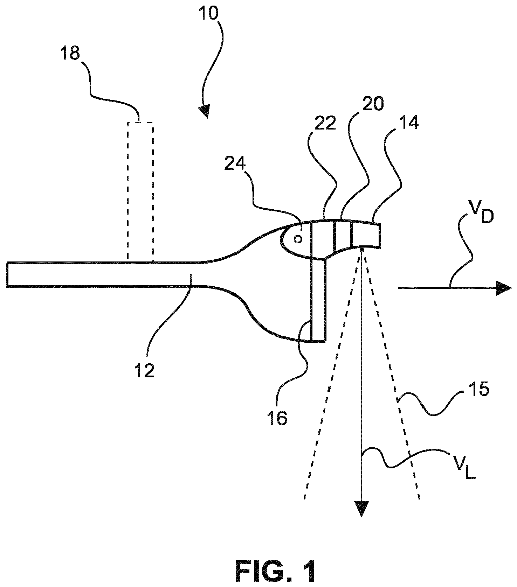

Fig. 1 schematically shows an example of a display device 10 for use during medical examinations. The display device 10 comprises a support structure 12 configured to be carried by a user as a head mounted gear. Further, a camera arrangement is provided comprising at least one optical camera 14 configured to provide live images of a region of interest in front of a user. Still further, also a display arrangement is provided comprising at least one projection surface 16 configured to display at least a part of the provided images of the region of interest to the user. The camera arrangement and the display arrangement are attached to the support structure. The at least one optical camera 14 has a line of vision *V_{L}.* The display arrangement is provided for a user's viewing direction *V_{D}* during use. The camera's line of vision *V_{L}* and the user's viewing direction *V_{D}* are arranged angular in relation to each other with an angle in a range of between 30° to 80°.

The support structure arrangement is also referred to as base, structure or frame. The display arrangement is also referred to as display. The camera arrangement is also referred to as camera.

The line of vision *V_{L}* is also referred to as central line of vision. The user's viewing direction *V_{D}* is also referred to as central line of vision.

In relation to the user's viewing direction being around the horizontal axis, e.g. with an inclination of up to +/- 15°, the camera is looking downwards in an inclined manner. In ergonomics, the user's forward viewing direction, when in an upright position, is usually considered to be slightly facing downwards, e.g. by 5° or 10°. In relation to that forward viewing direction, the camera is looking downwards in a much more inclined manner such that the camera is directed to an area directly in front of the user, which area can be reached by the hands of the user, while the user's head is in a comfortable position. In that position, for example, the user does not need to bend his/her neck of back.

As shown as an option, the support structure with the display and the camera is provided as a head mounted display. The display device can thus also be referred to as head mounted display device or head mounted display.

For example, the support structure may be provided with several head straps 18 such that the display device can be worn by a user safely and in a comfortable way.

In an example, a glasses frame structure for resting on the user's nose and behind the user's ears is provided.

For example, video glasses are provided. Displays may be arranged in front of the user's eye such that the view is non-distracted.

In an example, the head mounted display is provided as augmented reality glasses, where the user is non-encapsulated from reality.

In another example, a virtual reality headset is provided where one or two displays show the video stream images from reality. The user's view may be encapsulated by the headset.

In an example, the display receives the images from the camera. In an example, a data connection is provided at least between the camera and the display. In an example, a data processor is provided at least between the camera arrangement and the display arrangement.

In an example, not further shown, a light source is provided that is focused on the region of interest in the field of view.

In an example, the camera(s) see(s) the full surgical field, but only the part around the hands is displayed and magnified. For example, this provides a surgical loupe function.

The display device provides augmented reality to the user by projecting a camera view in the user's field of view. The user thus looks in a first direction but can see what he/she would see in a deviating second direction.

In Fig. 1, it is shown as an option that a hand detector 20 can be provided configured to recognize and detect at least a part of a user's hand in the region of interest in front of a user. The images displayed by the display are focused on the detected hand. The hand detector 20 is also referred to as hand recognizer.

In an example, the hand detector 20 is provided integrated with a data processing unit that provides the respective image analyzing and processing steps. In an example, the hand detector 20 is configured to detect the user's hand in the images provided by the camera.

In an example, the hand detector 20 is configured to detect the user's hand in the images with a location arrangement spatially tracking the user's hands and registration with the camera orientation.

In an option, the hand detector 20 is provided as a device detector. The device detector is configured to recognize and detect at least a part of a predetermined device in the region of interest in front of the user. The images displayed by the display are focused on the detected device. For example, this can be suitable in situations where the user's hands are rather interacting with a control unit of a device, such as for a surgical tool, and the field of interest is rather a tip of the surgical tool.

In Fig. 1, it is shown as an additional or alternative option that a magnifier 22 may be provided configured to magnify the images provided by the camera such that the display shows the region of interest in a magnified manner. The magnification is provided such that the displayed image is focused on the user's hands.

In an example, the magnifier is provided integrated with a data processing unit that provides the respective image analyzing and processing steps.

In an example, X-ray images of the region of interest are provided, and the display arrangement is configured to combine the X-ray images with the live images of the region of interest. In an example, in the displayed image, the X-ray images are overlaid with the live images of the region of interest.

In an example, hyperspectral images of the region of interest are provided, and the display arrangement is configured to combine the X-ray images with the live images of the region of interest. In an example, in the displayed image, the hyperspectral images are overlaid with the live images of the region of interest.

In an example, multiple cameras are provided to acquire image data for 3D images. For example, 3D recognition of the subject or an interventional device is provided.

In an example, hand detection (gesture control) or voice recognition is provided to allow panning and zooming of the camera. As a further option, in addition or alternatively, a location of the display arrangement is changed based on the gesture- or voice-control.

For example, a loupe function with a magnification of 3.5 times may be provided. This may be suitable for example for performing small tactical actions like stitching, cutting or wire maneuvers.

In an example, not further shown in detail, during use, the line of vision is inclined downwards to a larger extent than the user's viewing direction. Further, during use, the user's viewing direction is in a horizontal range of 25° inclined downwards from the horizontal, and 10° inclined upwards from the horizontal; and wherein the line of vision is in a range of 25° to 90° inclined downwards from the horizontal.

In Fig. 1, it is shown as an additional or alternative option that an angular adjuster 24 may be provided configured to adjust the angle between the line of vision and the user's viewing direction.

This allows adaption of a preferred posture of e.g. a surgeon in dependency of the respective examination. The term "examination" refers to any medical examination, investigation, operation or any other medically related action.

In an example, not further shown in detail, the at least one projection surface is at least partially transparent such that the user can also at least partly observe reality through the projection surface.

In an example, the transparency of the projection surface is adjustable. Thus, an adjustable dual reality view is provided.

In an example, not further shown in detail, the at least one projection surface is a display surface on which the images provided by the camera are presented. Further, for providing a live view of the situation along the viewing direction, further respective image data is provided and displayed on the display surface.

For the further image data, the same camera can be used, or a further camera is provided.

In an option, the hand detector 20 is provided in combination with the magnifier 22 and the angular adjuster 24. In another option, the hand detector 20 is provided in combination with the magnifier 22. In a still further option, the hand detector 20 is provided in combination with the angular adjuster 24. In a furthermore option, the magnifier 22 is provided in combination with the angular adjuster 24.

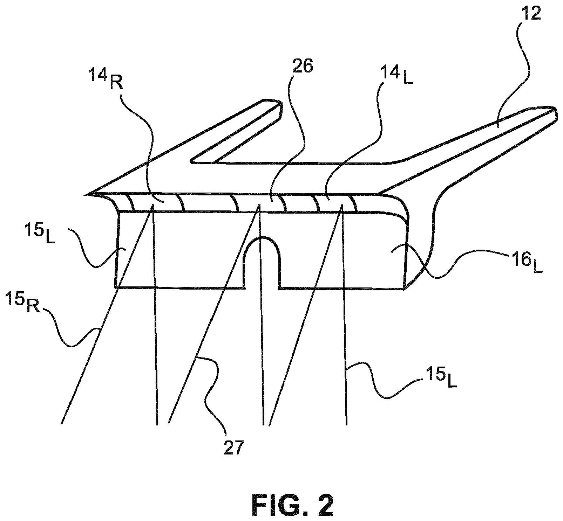

Fig. 2 shows an isometric view of a spectacle-like or glasses-like structure, i.e. a structure that can be worn by the user in front of his/her eyes and resting on the nose and behind/above the ears. Further support may also be provided such as head-mounted straps to temporarily secure and fix the device on the user in a comfortable manner.

Fig. 2 shows, as an option, the device provided as an example of a medical imaging system. The medical imaging system comprises an example of the display device 10, according to one of the examples above and below, and at least one additional imaging device providing further image data of the region of interest. The further image data is also shown on the display. As an option for the at least one additional imaging device, Fig. 2 comprises a hyperspectral camera 26 and a light source (not shown) suitable for hyperspectral imaging.

As an option, the hyperspectral camera 26 is attached to the display device 10.

As another option (not shown), the hyperspectral camera 26 is not attached to the display device 10 but carried otherwise by the user.

The light source suitable for hyperspectral imaging may also be arranged to be attached to the display device 10. However, it is also provided that the light source suitable for hyperspectral imaging is arranged independently, e.g. carried by the user or also not carried by the user.

Hyperspectral imaging enables visualization of light beyond the human visible spectrum which is suitable e.g. in the surgical field. As an example, hyperspectral imaging enables indocyanine green (ICG) dye visualization, lymph node mapping and tumor identification. Thus, hyperspectral imaging provides enhanced anatomical information.

In another example, not shown and provided in addition or alternatively to the hyperspectral imaging, the at least one additional imaging device is an X-ray imaging device that comprises an X-ray source and an X-ray detector. The X-ray imaging device may be arranged separately to the head mounted gear and the image data can be supplied to the display device by a wire connection or a wireless connection. The X-ray imaging device provides fluoroscopy X-ray images.

Fig. 2 also shows, as another option for the display device 10, an example where the display arrangement comprises a first optical camera 14_{L} and a second optical camera 14_{R} and a first projection surface 16_{L} and a second projection surface 16_{R}. The first optical camera 14_{R} and the second optical camera 14_{L} are distanced to each other and are configured to provide first and second live images of the region of interest. The respective two camera views are indicated with reference numerals 15_{L} and 15_{R}. The first projection surface 16_{R} and the second projection surface 16_{L} are distanced such that the first projection surface is assigned to be viewed by a user's first eye and that the second projection surface is assigned to be viewed by a user's second eye such that the user is provided with a stereoscopic view on the region of interest. This provides further detailed knowledge to the user about the spatial situation.

In an option, the hand detector 20, the magnifier 22, the angular adjuster 24, the stereo camera/display arrangement and the hyperspectral camera 26 and light source arrangement are provided in combination.

In another option, the hand detector 20, the magnifier 22, the stereo camera/display arrangement and the hyperspectral camera 26 and light source arrangement are provided in combination.

In a further option, the hand detector 20, the magnifier 22 and the hyperspectral camera 26 and light source arrangement are provided in combination.

In a still further option, the hand detector 20, the magnifier 22 and the stereo camera/display arrangement are provided in combination.

In a furthermore option, the magnifier 22, the stereo camera/display arrangement and the hyperspectral camera 26 and light source arrangement are provided in combination.

In a yet further option, the hand detector 20, the magnifier 22, the angular adjuster 24 and the hyperspectral camera 26 and light source arrangement are provided in combination.

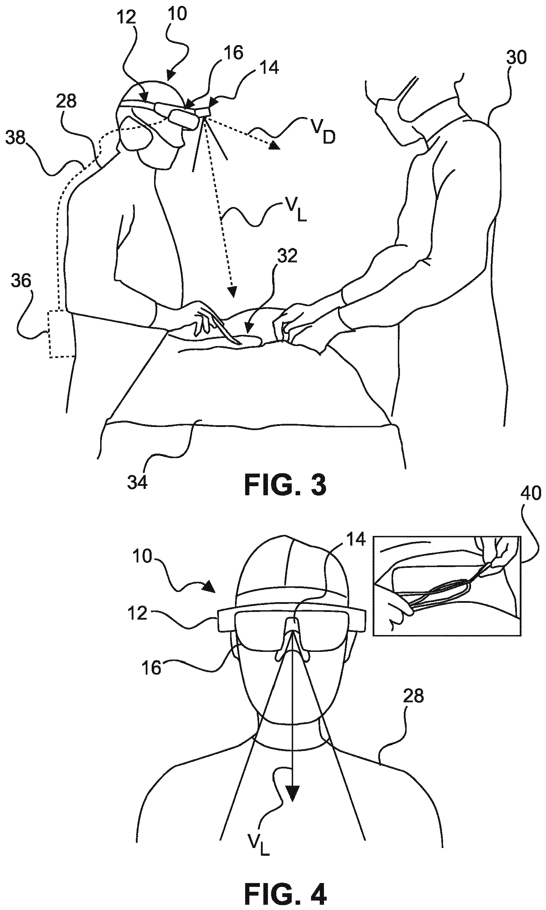

Fig. 3 shows an exemplary scenery in an operation room of a hospital during a medical examination. A first user 28, for example a surgeon, and a second user 30, for example an assisting staff member, are shown standing on two sides of a subject support, focusing on a region of interest 32 of a subject 34. As an example, the first user is operating an interventional device while the second user is assisting in this task.

The first user is using an example of the display device 10. In another example, not shown, also the second use is using an example of the display device 10.

In Fig. 3 it is shown as an option that the display arrangement comprises a light source 36 and a light transmitter 38. The light source 36 is provided remote from the support structure and is configured to be carried by the user at the hip, shoulder, back and/or arm. As an option it is shown that the user carries the light source 36 at the hip, for example by a not shown belt or the like. The light transmitter 38 optically couples the light source 36 with the projection surface. The head-mounted support can thus be reduced to a minimum and user comfort further increased.

The separate belt, or pouch option, even though the belt is not shown, can also be used to reduce the weight of the headset and can contain other parts such as battery, computing unit or wireless antennas or other interfaces, to further reduce the weight of the headmounted gear.

Fig. 4 shows a front view of a user wearing an example of the display device 10. Even though the user has his/her had in an upright position, in which the user looks forward in a more or less horizontal way, the camera(s) is(are) looking downwards and via the display arrangement the user is thus provided with the respective visual information. As an option, the visual information may comprise an enlarged, i.e. magnified view of the area in front of the user in which area the user has his/her hands for interaction with the subject. A frame 40 indicates the respective projection of the magnified part of the area provided to the user by the display arrangement.

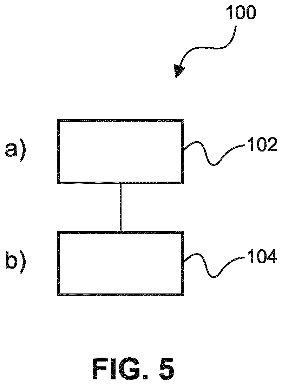

Fig. 5 shows basic steps of an example of method 100 for providing visual information during medical examination. The method 100 comprises the following steps:
- In a first step 102, also referred to as step a), live images of a region of interest in front of a user are acquired by at least one optical camera of a camera arrangement.
- In a second step 104, also referred to as step b), at least a part of the provided images of the region of interest is displayed to the user by at least one projection surface of a display arrangement. The camera arrangement and the display arrangement are attached to the support structure. The support structure is carried by a user as a head mounted gear. The at least one optical camera has a line of vision, and the display arrangement is provided for a user's viewing direction during use. The camera's line of vision and the user's viewing direction are arranged angular in relation to each other with an angle in a range of between 30° to 80°.

In an example, not further shown, voice commands can be used to enable or disable the hand tracking feature. As an option, instead of the hand tracking feature (or after the hand tracking feature has been disabled), the optical cameras are used to keep the zoom/pan of the camera at the region of interest.

In an example, the optical cameras include an additional structured light / 3D optical camera.

As a further option, optical markers can be placed on the patient, such as skin markers. These markers can be used to determine the location of surgery. As an example, this is the center of the optical markers, but it is also possible that a wireless connection to an optical navigation system is used to determine the location of surgery in relationship to the optical markers.

In an addition, a graphic may be visualized where the surgical planned path in relation to the optical markers is located, so the surgeon knows where to be active.

As an option, a wireless connection to other computer systems, such as an optical navigation system, can be used to visualize the complete or only a part of the display or user interface of these systems on the headset.

Still further, voice commands can be used to influence where the camera image is shown on the display and whether it is turned on/off. This may also affect the 'darkening' of the part of the display that is relevant for the display.

In an example, MEMS sensors (accelerometers, gyroscopes or the like) are used to zoom/pan the camera image to prevent unneeded movement of the zoomed area when the head is moved.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it, which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A display device (10) for use during medical examinations, the display device comprising:
- a support structure (12);
- a camera arrangement; and
- a display arrangement;
wherein the camera arrangement and the display arrangement are attached to the support structure; and the support structure is configured to be carried by a user as a head mounted gear;
wherein the camera arrangement comprises at least one optical camera (14) configured to provide live images of a region of interest in front of a user;
wherein the display arrangement comprises at least one projection surface (16) configured to display at least a part of the provided images of the region of interest to the user;
wherein the at least one optical camera has a line of vision (*V_{L}*), and the display arrangement is provided for a user's viewing direction (*V_{D}*) during use; and
wherein the camera's line of vision and the user's viewing direction are arranged angular in relation to each other with an angle in a range of between 30° to 80°.

2. Display device according to claim 1, wherein a hand detector (20) is provided configured to recognize and detect at least a part of a user's hand in the region of interest in front of a user; and
wherein the images displayed by the display are focused on the detected hand.

3. Display device according to claim 1 or 2, wherein a magnifier (22) is provided configured to magnify the images provided by the camera such that the display shows the region of interest in a magnified manner;
wherein the magnification is provided such that the displayed image is focused on the user's hands.

4. Display device according to claim 1, 2 or 3, wherein, during use, the line of vision is inclined downwards to a larger extent than the user's viewing direction; and
wherein, during use, the user's viewing direction is in a horizontal range of 25° inclined downwards from the horizontal, and 10° inclined upwards from the horizontal; and wherein the line of vision is in a range of 25° to 90° inclined downwards from the horizontal.

5. Display device according to one of the preceding claims, wherein an angular adjuster (24) is provided configured to adjust the angle between the line of vision and the user's viewing direction.

6. Display device according to one of the preceding claims, wherein the at least one projection surface is at least partially transparent such that the user can also at least partly observe reality through the projection surface.

7. Display device according to one of the preceding claims, wherein the at least one projection surface is a display surface on which the images provided by the camera are presented; and
wherein, for providing a live view of the situation along the viewing direction, further respective image data is provided and displayed on the display surface.

8. Display device according to one of the preceding claims, wherein the support structure with the display and the camera is provided as a head mounted display.

9. Display device according to one of the preceding claims, wherein the display arrangement comprises:
- a light source (36); and
- a light transmitter (38);
wherein the light source is provided remote from the support structure and is configured to be carried by the user at at least one of the group of hip, shoulder, back and arm; and
wherein the light transmitter optically couples the light source with the projection surface.

10. A medical imaging system comprising:
- a display device (10) according to one of the preceding claims;
- at least one additional imaging device providing further image data of the region of interest;
wherein the further image data is also shown on the display.

11. Imaging system according to claim 10, wherein the at least one additional imaging device comprises a hyperspectral camera (26) and a light source suitable for hyperspectral imaging; and
wherein the hyperspectral camera is attached to the display device.

12. Imaging system according to claim 10 or 11, wherein the at least one additional imaging device is an X-ray imaging device that comprises an X-ray source and an X-ray detector.

13. A method (100) for providing visual information during medical examination, comprising the following steps:
a) acquiring (102) live images of a region of interest in front of a user by at least one optical camera of a camera arrangement; and
b) displaying (104) at least a part of the provided images of the region of interest to the user by at least one projection surface of a display arrangement;
wherein the camera arrangement and the display arrangement are attached to the support structure; and the support structure is carried by a user as a head mounted gear;
wherein the at least one optical camera has a line of vision, and the display arrangement is provided for a user's viewing direction during use; and wherein the line of vision and the user's viewing direction are arranged angular in relation to each other with an angle in a range of between 30° to 80°.

14. A computer program element for controlling an apparatus according to one of the claims 1 to 12, which, when being executed by a processing unit, is adapted to perform the method steps of claim 13.

15. A computer readable medium having stored the program element of claim 14.
